# EUROPÄISCHE PATENTANMELDUNG

(11) **EP 1 070 488 A1**
(43) Veröffentlichungstag der Anmeldung: **24.01.2001**
(21) Anmeldenummer: 00810539.7
(22) Anmeldetag: 20.06.2000
(51) Int. Cl.: A61F 2/30, B24C 1/04

(54) **Verfahren zur Herstellung einer Oberflächenstruktur, insbesondere auf einem chirurgischen Implantat**

(30) Priorität: 19.07.1999 EP 99810651
(71) Anmelder: SULZER INNOTEC AG, CH-8401 Winterthur (CH)
(72) Erfinder: Straub, Werner, 8477 Oberstammheim (CH); Sprecher, Christoph, 7260 Davos Dorf (CH); Peters, John Antony, Dr., 8404 Winterthur (CH); Windler, Markus, 8354 Hofstetten (CH)
(74) Vertreter: Sulzer Management AG

(57) **Zusammenfassung**

Bei dem Verfahren zur Herstellung einer Oberflächenstruktur wird Material mittels eines Flüssigkeitsstrahls (1) abgetragen. Der Strahl (1) wird aus einer Düse (10) unter hohem Druck (p) abgegeben. Dabei wird eine Abtragsstelle (3) auf einer zu strukturierenden Oberfläche (20a) eines Substrats (20) unter Erzeugung einer vorgegebenen Makrotopographie (2') oder einer weitgehend planen Oberfläche gesteuert bewegt, nämlich durch Bewegen der Düse und/oder des Substrats. Das Substrat ist insbesondere Teil eines chirurgischen Implantats. Die Flüssigkeit des Hochdruckstrahls (1) wird bei einem vorgegebenen Durchmesser d der Düse mit einem genügend hohen Druck p abgegeben, so dass durch den Materialabtrag eine lineare Spur (2) mit quasi-fraktaler Mikrotopographie (4) erzeugt wird. Dabei ist die Spurbreite D mindestens doppelt so gross wie d. Für p und d sind Werte in folgenden Bereichen vorgesehen:
100 bar < p < 3000 bar und 0.1 mm < d < 10 mm;
oder p > 3000 bar und d > 0.03 mm.

## Beschreibung

Die Erfindung betrifft ein Verfahren zur Herstellung einer Oberflächenstruktur, insbesondere auf einem chirurgischen Implantat, gemäss Oberbegriff von Anspruch 1 sowie eine Anlage zum Durchführen des Verfahrens und Anwendungen des Verfahrens.

Aus der EP-A 0 417 034 (= P.6300) ist ein Implantat bekannt, das Verankerungsflächen für Knochengewebe aufweist. Die Verankerungsfläche ist dabei mittels eines Verfahrens hergestellt, bei dem geometrische Raumformen unter Verwendung eines Hochdruckflüssigkeitsstrahls erzeugt werden. Um mit dem Flüssigkeitsstrahl eine geeignete Tiefenwirkung erzielen zu können, sind der Flüssigkeit abrasive Partikel beigemischt. Diese Partikel, die spurenweise in den erzeugten Vertiefungen zurückbleiben, müssen für den menschlichen Körper verträglich sein. Bei diesem bekannten Verfahren ist vorausgesetzt, dass mit einem Hochdruckstrahl, der linear über ein Substrat geführt wird, eine nutförmige Abtragsspur im Substrat entsteht, deren Breite weitgehend gleich gross wie der Strahldurchmesser oder der Durchmesser der verwendeten Düse ist.

Aufgabe der Erfindung ist es, ein Verfahren zur Herstellung einer Oberflächenstruktur durch Materialabtrag mittels eines Flüssigkeitsstrahls zu schaffen, bei der eine Abtragsspur entsteht, die aufgrund der erzeugten Mikrostruktur oder Mikrotopographie besonders vorteilhaft bezüglich physiologischen Vorgängen ist. Unter solchen physiologischen Vorgängen werden beispielsweise das An- und Einwachsen von Knochengewebe bei einer Hüftgelenkprothese oder eine elektrophysiologische Einwirkung auf ein zu stimulierendes Gewebe beispielsweise durch eine Herzschrittmacher-Elektrode verstanden. Diese Aufgabe wird durch das im Anspruch 1 definierte Verfahren gelöst.

Bei dem Verfahren zur Herstellung einer Oberflächenstruktur wird Material mittels eines Flüssigkeitsstrahls abgetragen. Der Strahl wird aus einer Düse unter hohem Druck abgegeben. Dabei wird eine Abtragsstelle auf einer zu strukturierenden Oberfläche eines Substrats unter Erzeugung einer vorgegebenen Makrotopographie oder einer weitgehend planen Oberfläche gesteuert bewegt, nämlich durch Bewegen der Düse und/oder des Substrats. Das Substrat ist insbesondere Teil eines chirurgischen Implantats. Die Flüssigkeit des Hochdruckstrahls wird bei einem vorgegebenen Durchmesser d der Düse mit einem genügend hohen Druck p abgegeben, so dass durch den Materialabtrag eine lineare Spur mit quasi-fraktaler Mikrotopographie erzeugt wird. Dabei ist die Spurbreite D mindestens doppelt so gross wie d. Für p und d sind Werte in folgenden Bereichen vorgesehen:
100 bar < p < 3000 bar und 0.1 mm < d < 10 mm;
oder p > 3000 bar und d > 0.03 mm.

Das erfindungsgemässe Verfahren kann auch bei Oberflächenbearbeitungen vorteilhaft sein, bei denen nichtmedizinische Gegenstände bearbeitet werden:
a) Erzeugung von Strukturen an homogenen, quasiisotropen Werkstoffen, um beispielsweise eine Welligkeit oder einen Orangenhauteffekt zu erzielen;
b) Strukturierung von inhomogenen Werkstoffen, wobei unterschiedliches Verhalten bezüglich Löslichkeit oder Erosionsraten genutzt wird.

Die abhängigen Ansprüche 2 bis 6 beziehen sich auf vorteilhafte Ausführungsformen des erfindungsgemässen Verfahrens. Gegenstand der Ansprüche 7 und 8 ist eine Anlage zum Durchführen des Verfahrens. Die Ansprüche 9 bis 11 betreffen Anwendungen des Verfahrens.

Nachfolgend wird die Erfindung anhand der Zeichnungen erläutert. Es zeigen:
- Fig. 1: einen Materialabtrag in Form von geradlinigen Abtragsspuren, welcher mit dem erfindungsgemässen Verfahren mittels eines Hochdruck-Flüssigkeitsstrahls durchgeführt wird,
- Fig. 2: ein Ergebnis des erfindungsgemässen Verfahrens,
- Fig. 3: ein Profil einer erfindungsgemäss bearbeiteten Oberfläche,
- Fig. 4: Teilergebnisse einer Fraktalanalyse zum Profil der Fig. 3,
- Fig. 5: Diagramme zu Fraktalanalysen,
- Fig. 6: einen Düsenkopf mit Kollimatorrohr,
- Fig. 7: ein Profil einer asymmetrischen Abtragsspur,
- Fig. 8: eine Mittellinie zu einer mäandrischen Abtragsspur,
- Fig. 9: Mittellinien zu Abtragsspuren, die mit drei Scharen von sich kreuzenden, geradlinigen Spuren hergestellt sind, und
- Fig. 10: eine Anlage zum Durchführen des erfindungsgemässen Verfahrens.

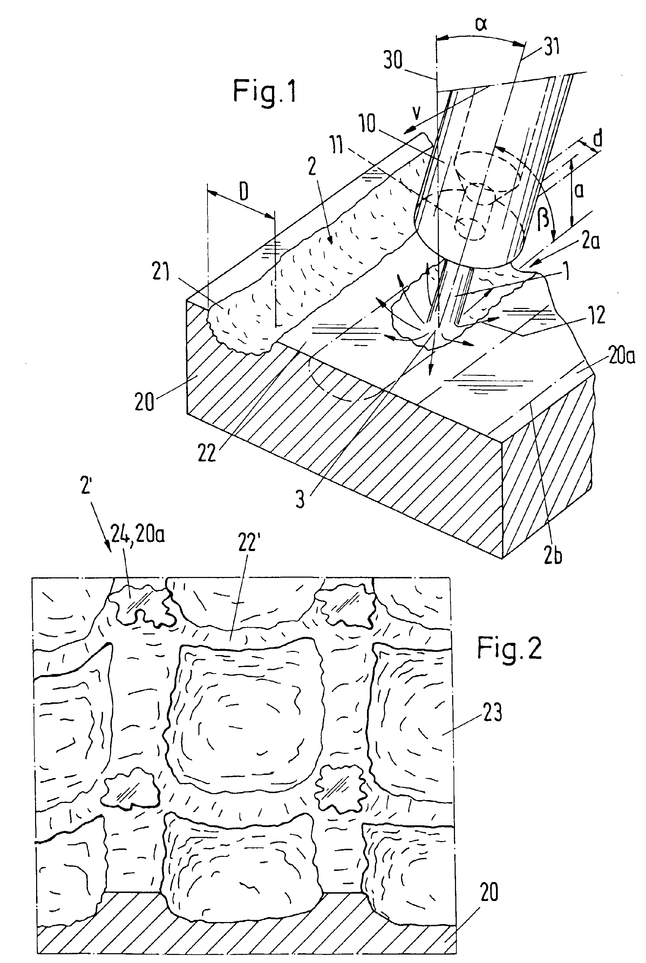

Bei einem Materialabtrag mittels eines Hochdruck-Flüssigkeitsstrahls 1, wie er in Fig. 1 dargestellt ist, werden geradlinige, nutförmige Abtragsspuren 2 auf einem Substrat 20 erzeugt. Der Strahl 1 tritt aus einer Austrittsöffnung 11 einer Düse 10 aus, die mit einer Geschwindigkeit v in Richtung der zu erzeugenden Spur 2a bewegt wird. Er trifft in einer Einwirkzone 3 (Abtragsstelle) auf der Oberfläche 20a des Substrats 20 auf. Mit strichpunktierten Linien 2b sind Umrisse von Abtragsspuren angegeben, die anschliessend beim Fortführen des Abtragsverfahrens entstehen werden.

Eine Gerade 31, die als Mittellinie die Richtung des Strahls 1 angibt, ist in der Regel gegenüber einer Senkrechten 30 zur Oberfläche 20a um einen Winkel α geneigt. Der Winkel α beträgt bei der Bearbeitung von verschiedenen metallischen Legierungen mit Vorteil rund 30°; der Materialabtrag ist für diesen Winkel besonders wirkungsvoll. Die Austrittsöffnung 11 der Düse 10 hat einen Durchmesser d; sie befindet sich in einem Abstand a von der Einwirkzone 3. Der Durchmesser d, nämlich der Düsendurchmesser, ist im wesentlichen auch der Durchmesser des austretenden Strahls 1.

Als Flüssigkeit für den Hochdruckstrahl wird mit Vorteil Wasser verwendet, das insbesondere bei der Herstellung von Oberflächenstrukturen auf chirurgischen Implantaten aufbereitet sein soll, wobei eine Aufbereitung Filtrieren, Entmineralisieren und Zugeben von Zusatzstoffen umfassen kann. Es ist aber auch möglich, eine Flüssigkeit zu verwenden, die ätzend auf das Substrat einwirkt. Man kann auch Feststoffpartikel in der Flüssigkeit suspendieren, die beim Auftreffen in der Einwirkzone 3 eine abrasive Wirkung entfalten. Bei chirurgischen Implantaten kann es vorteilhaft sein, ohne solche Feststoffpartikel zu arbeiten, so dass keine Kontamination der zu bearbeitenden Oberfläche durch Rückstände der Feststoffpartikel entsteht. Allerdings lassen sich biokompatible Feststoffpartikel verwenden, die inert, bioaktiv oder degradabel sind. Ein bioaktiver Stoff ist beispielsweise Pentacalciumphosphat (Hydroxylapatit) oder eine andere Calcium-Phosphor-Verbindung, durch die ein Knochenwachstum gefördert wird.

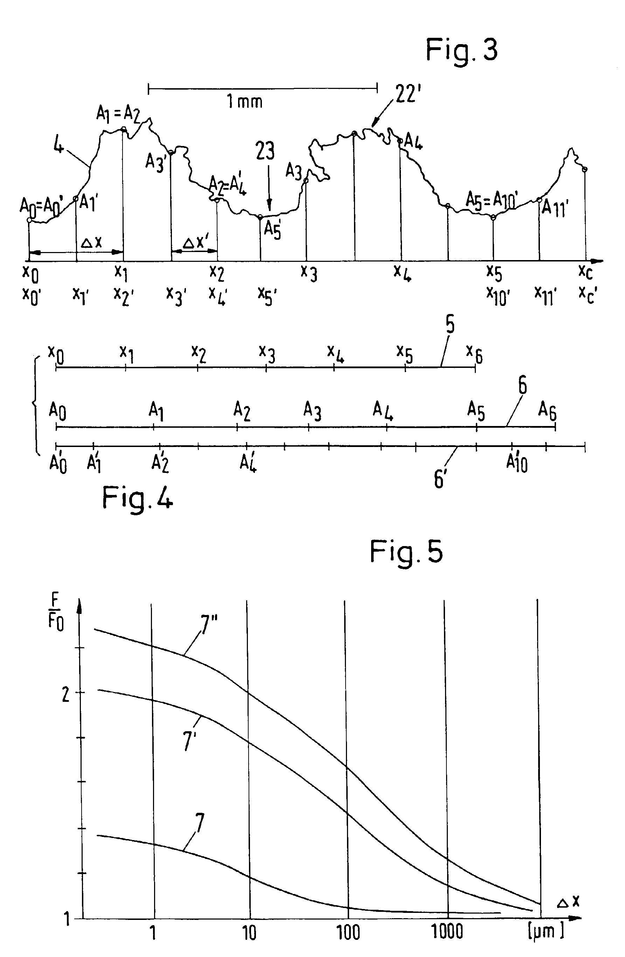

Der Strahl 1 schlägt in der Einwirkzone 3 Partikel aus der Oberfläche 20a, die seitlich weggespült werden. Die seitlich abströmende Flüssigkeit 12 entfernt unter einem Mitwirken der freigesetzten Partikel weiteres Material aus dem Substrat 20. Es entsteht so eine Nut 2 der Breite D, die mindestens doppelt so gross wie der Düsendurchmesser d ist. Die so freigelegte Oberfläche 21 der Nut 2 weist eine zerklüftete Mikrotopographie auf, die als "quasi-fraktal" bezeichnet werden kann (vgl. nachfolgende Ausführungen zu Fraktalanalysen im Zusammenhang mit den Figuren 3 bis 5). Liegen die Geraden 30 und 31 mit dem Vektor der Geschwindigkeit v in einer gemeinsamen Ebene, so ergibt sich eine Form der Nut 2, deren Querschnitt symmetrisch ist.

Versuche ergaben folgende Resultate:
a) Bei einem Düsendurchmesser d = 0.13 mm, einem Strahldruck p von 3000 bar und einem Vorschub v von 250 mm/min entstand eine gut ausgebildete Spur mit einer Breite D, die zwischen einem Minimalwert von Dₘᵢₙ = 0.30 mm und Dₘₐₓ = 0.60 mm schwankt. Das Verhältnis Dₘᵢₙ/d ist somit 2.31.
b) Bei d = 0.18 mm, p = 1500 bar und v = 1500 mm/min ergab sich Dₘᵢₙ = 0.50 mm und Dₘₐₓ = 0.70 mm; folglich Dₘᵢₙ/d = 2.78.
c) Bei d = 0.40 mm, p = 3500 bar und v = 100 mm/min ergab sich Dₘᵢₙ = 0.90 mm und Dₘₐₓ = 1.10 mm; folglich Dₘᵢₙ/d = 2.25.

Der Hochdruckstrahl 1 wird in der Regel kontinuierlich auf das Substrat 20 aufgespritzt. Es ist aber auch möglich, einen gepulsten Strahl 1 zu verwenden.

Bei dem in Fig. 1 dargestellten Verfahren entsteht eine Makrotopographie, die durch parallele, geradlinige Abtragsspuren 2 gebildet ist. Zwischen benachbarten Spuren 2 wird ein Grat 22 stehen gelassen. Die einzelnen Spuren 2 können aber auch sehr dicht nebeneinander angeordnet werden, so dass praktisch keine Grate 22 mehr erkennbar sind: Es entsteht eine weitgehend plane Fläche mit einer quasi-fraktalen Mikrotopgraphie.

Auf eine erste Schar von geradlinigen Spuren 2, wie sie mit einer Oberflächenbearbeitung gemäss Fig. 1 herstellbar ist, lässt sich eine zweite Schar aufbringen, die kreuzweise zu der ersten angeordnet wird. Das Resultat 2' einer auf diese Art erzeugten Makrotopographie ist in Fig. 2 dargestellt (nach einem REM-Bild gezeichnet). Die erzeugte Reliefstruktur 2' ist schachbrettartig. Die einzelnen Felder sind Mulden 23, die durch Kämme 22' gegeneinander abgegrenzt sind. In den Eckpunkten dieser Felder sind Inseln 24 mit Resten der urspünglichen Oberfläche 20a des Substrats 20 sichtbar. Das bearbeitete Material ist eine Titanlegierung (Ti-6Al-4V: Titan mit 6 Gew-% Aluminium und 4 Gew-% Vanadium). Der Abstand zwischen den Eckpunkten beträgt 1 mm. Es wurde eine Düse verwendet, deren Durchmesser 0.18 mm betrug. Als Flüssigkeit wurde Wasser bei einem Druck von 3500 bar verwendet.

Eine Rasterstruktur, wie sie in Fig. 2 dargestellt ist, lässt sich auch rationeller mit einer Mehrfachdüse herstellen, bei der mit einer Anzahl von Düsenöffnungen, die auf einer Reihe von äquidistanten Punkten angeordnet sind, eine Schar paralleler Spuren 2 simultan erzeugbar ist.

Fig. 3 zeigt das Profil eines Querschnitts durch die in Fig. 2 gezeigte Reliefstruktur 2'. Das Profil ist als Kurve 4 über einer x-Achse dargestellt. Die x-Achse wird durch Punkte x₀, x₁, ... oder x₀', x₁', ... gleichmässig in Intervalle Δx bzw. Δx' (= ½ Δx) unterteilt. Diesen Unterteilungen entsprechen Punkte A₀, A₁, ... bzw. A₀', A₁', ... Es werden die Längen der Streckenzüge A₀, A₁, ... bzw. A₀', A₁', ... bestimmt und verglichen. Ein graphischer Vergleich ist in Fig. 4 gegeben. Die Gesamtstrecke 5 stellt die Länge auf der x-Achse dar; die Gesamtstrecken 6 bzw. 6' die Längen der Streckenzüge A₀, A₁, ... bzw. A₀', A₁', ... Offensichtlich sind diese Längen um so grösser, je feiner die Unterteilung Δx der x-Achse ist. Diese Länge gibt als Funktion von Δx ein Mass für die Fraktalität des Profils 4. Nimmt diese Funktion mit kleiner werdendem Δx kontinuierlich zu und zwar bis zu einem minimalen Δx, das aus messtechnischen Gründen nicht unterschritten werden kann, so wird hier für die Mikrotopographie des vermessenen Profils die Bezeichnung "quasi-fraktal" verwendet. Bei einer gängigen Fraktalanalyse wird etwas anders vorgegangen: Die Streckenabschnitte zwischen benachbarten Punkten Aᵢ werden als konstante Grössen vorgegeben (so dass die Intervalle Δx variabel sind).

Das Diagramm der Fig. 5 zeigt Resultate einer entsprechenden Fraktalanalyse für drei Proben aus Reintitan cpTi (commercial pure Ti). Bei dieser Analyse wurde statt eines Anwachsens von Längen die Vergrösserung einer Oberfläche F bestimmt, deren Flächenelemente Dreiecke sind. Die Ergebnisse für F sind - auf die Grundfläche F₀ bezogen - im Diagramm eingetragen. Die drei Kurven 7, 7' und 7" gewann man anhand von gewalzten Proben aus Ti, die mittels einer Schar paralleler Spuren 2 aufgerauht wurden (Versatz benachbarter Spuren: b), wobei die Verfahrensparameter folgendermassen gewählt wurden: d = 0.25 mm, a = 15 mm, b = 0.20 mm, p = 3500 bar, v = 1.25 m/min (Kurve 7"), v = 2.00 m/min (Kurve 7') und v = 5.00 m/min (Kurve 7).

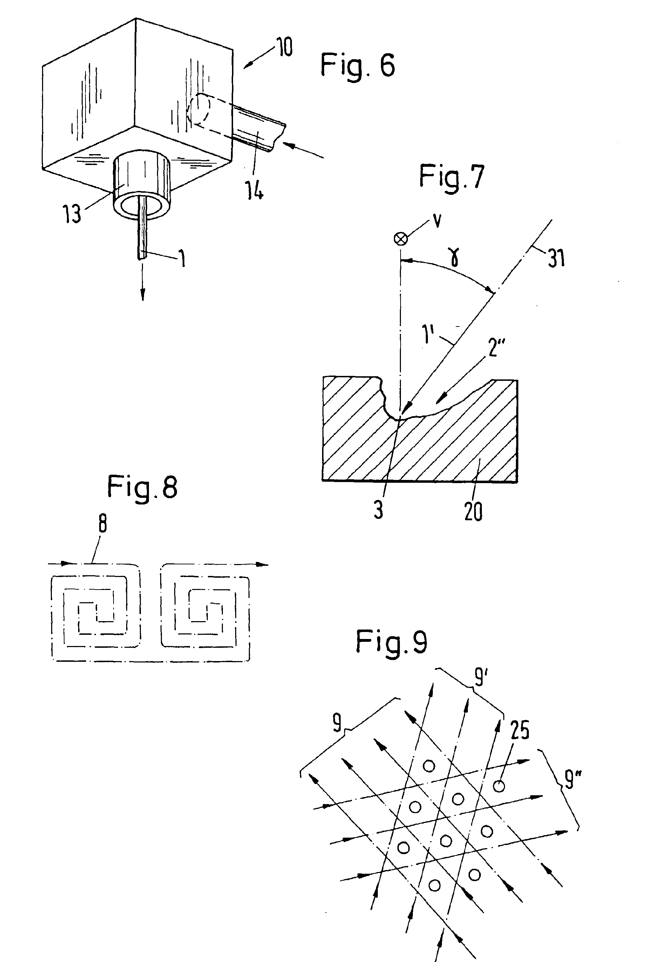

Der Abstand a zwischen der Austrittsöffnung 11 der Düse 10 und der Einwirkzone 3 kann zwischen rund 0.1 - 20 mm betragen. Der Winkel β zwischen dem Flüssigkeitsstrahl 1 (β = 90° - α) und der erzeugten Abtragsspur 2 kann 90° (d. h. α = 0°) betragen, wird aber vorzugsweise kleiner als 90° gewählt, um so einen wirkungsvolleren Materialabtrag zu erhalten. Da der Düsenkopf bei gebräuchlichen Einrichtungen relativ voluminös ausgebildet ist, bestehen Schwierigkeiten beim Einstellen der Strahlrichtung, wenn der Strahl unter einem spitzen Winkel auf dem Substrat 20 auftreffen soll. Anbringen eines Kollimatorrohrs 13 vor der Austrittsöffnung 11 - siehe Fig. 6 - ermöglicht es, Winkel einzustellen, die von der Senkrechten 30 um mindestens 30° abweichen. Das Kollimatorrohr 13 schirmt den Strahl 1 von der Umgebung ab, so dass der Abstand a zwischen Austrittsöffnung 11 und Einwirkzone 3 grösser als die genannte Distanz von 20 mm sein kann, ohne dass es zu einer wesentlichen Beeinträchtigung der Qualität des Strahls 1 kommt. In Fig. 6 ist zusätzlich ein Anschlussrohr 14 gezeigt, durch das die Hochdruckflüssigkeit in die Düse 10 eingespeist wird.

Wird der Strahl 1 gemäss Pfeil 1' in Fig. 7 auf das Substrat 20 gerichtet, so dass die Strahlmittellinie 31 nicht in der durch die Senkrechte 30 und die Geschwindigkeit v aufgespannten Ebene liegt, so entsteht eine Spur 2" mit einem asymmetrischen Querschnitt. Bei dem gezeigten Beispiel schliessen die Geraden 31 und 30 - in Richtung der Geschwindigkeit v gesehen - bei der Einwirkzone 3 einen Winkel γ ein.

Die Abtragsspur 2 muss nicht wie in den Beispielen der Figuren 1 und 2 geradlinig sein. Sie kann beispielsweise auch - wie in Fig. 8 dargestellt - entlang einer mäanderförmigen Kurve 8 geführt werden. Ist die Kurve 8 die Mittellinie einer genügend breiten Abtragsspur 2, so dass sich keine Grate zwischen benachbarten Kurvenstücken ausbilden, so ergibt sich durch den Mäander ein relativ breiter Abtragsstreifen bei einem einmaligen Durchgang.

In der Regel kann jede Abtragsspur durch ein einmaliges oder mehrfaches Wiederholen des Abtragdurchgangs vertieft werden. Wenn allerdings bei einem ersten Durchgang die zu bearbeitende Oberfläche weitgehend intakt bleibt, führen in der Regel weitere Durchgänge zu keiner Ausbildung einer brauchbaren Abtragsspur. Es kann jedoch sein, dass bei einem ersten Durchgang entlang der Abtragskurve einzelne kraterartige Vertiefungen entstehen. Diese Vertiefungen können sich dann bei weiteren Durchgängen vergössern und schliesslich zu einer vollständig ausgehobenen Nut führen.

Während in Fig. 2 eine Makrotopographie dargestellt ist, die mit zwei Scharen von sich kreuzenden, geradlinigen Spuren hergestellt ist, zeigt Fig. 9 ein entsprechendes Muster von Abtragsspuren, bei dem die Oberflächenstrukturierung mit drei Scharen 9, 9' und 9" gebildet wird. Es sind nur die Mittellinien zu den Abtragsspuren dargestellt. Mit kleinen Kreisen 25 sind die Stellen gekennzeichnet, an denen inselartige Erhebungen - entsprechend zu den Inseln 24 in Fig. 2- stehen bleiben können.

Das erfindungsgemässe Verfahren kann auch mit weiteren Verfahren kombiniert werden: in einem ersten oder zweiten Schritt kann ein weiteres Verfahren zu einer Oberflächenbehandlung, beispielsweise ein Sandstrahl- oder ein Beschichtungsverfahren, angewendet werden. Im Fall eines Sandstrahlverfahrens wird dieses vorzugsweise in dem ersten Schritt angewendet, um beim zweiten Schritt, der mit einer partikelfreien Flüssigkeit durchgeführt wird, allfällige durch Sandpartikel verursachteVerunreinigungen eliminieren zu können.

Eine besonders vorteilhafte Anwendung des erfindungsgemässen Verfahrens ist die Oberflächenstrukturierung bei einer Gelenkprothese, die für eine zementlose Implantation vorgesehen ist. Bei dieser Prothese muss die Schaftoberfläche geeignet strukturiert werden, damit ein Knochenwachstum gefördert wird. Die beschriebene quasi-fraktale Mikrotopograpie ist dabei besonders geeignet. Eine Makrotopographie, wie sie in Fig. 2 abgebildet ist, ermöglicht zusätzlich eine gute Verankerung während der Anfangsphase, während der eine Verbindung zwischen Knochen und Prothese noch sehr schwach ausgebildet ist. Eine entsprechende Anwendung ist auch bei Dentalimplantaten vorteilhaft, bei denen das Implantat in einem Kiefer durch ein Knochenwachstum einen stabilen Halt gewinnen muss. Auch bei Gelenkprothese, die unter Verwendung eines Zements implantiert werden, kann das erfindungsgemässe Verfahren angewendet werden, um Zonen der Oberfläche aufzurauhen, auf denen der Zement haften muss.

Eine weitere vorteilhafte Anwendung des erfindungsgemässen Verfahrens betrifft elektrophysiologische Elektroden, beispielsweise bei Herzschrittmachern oder Defibrillatoren. Bei solchen Elektroden müssen Pole an deren Oberflächen strukturiert werden, um - dank einer grossen spezifischen Oberfläche - eine wirkungsvolle Energieübertragung von dem Pol auf ein physiologisch zu beeinflussendes Gewebe zu erhalten. Wie Spannungsmessungen gezeigt haben, ist die quasi-fraktale Mikrotopograpie auch hier besonders gut geeignet.

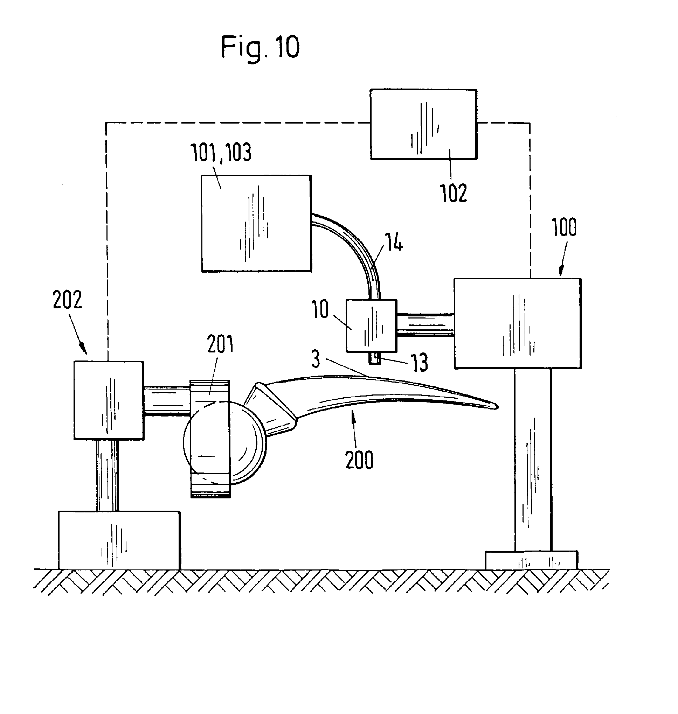

In Fig. 10 ist schematisch eine Anlage zum Durchführen des erfindungsgemässen Verfahrens dargestellt. Sie umfasst folgendes: Mittel zum Erzeugen einer Relativgeschwindigkeit zwischen dem zu bearbeitenden Körper 200 (hier eine Hüftgelenkprothese) und der Abtragsstelle 3 auf dem Körper, nämlich eine Einrichtung 100 zum gesteuerten Bewegen der Düse 10 und eine Einrichtung 102 mit einer gesteuert bewegbaren Halterung 201 für den Körper 200; ferner eine elektronische, programmierbare Steuereinrichtung 102 für die Einrichtungen 100 und 202. Die Düse 10 oder der Körper 200 können auch jeweils in einer raumfesten Position gehalten werden. Die Düse 10 (mit dem Kollimatorrohr 13) ist über eine flexible Druckleitung 14 mit einer Teilanlage verbunden, die folgendes umfasst oder umfassen kann: eine Hochdruckpumpe 101 für die Erzeugung des nicht dargestellten Flüssigkeitsstrahls und eine Einrichtung 103 zum Aufbereiten der Flüssigkeit, insbesondere zu einer Filtrierung und/oder Entmineralisierung der Flüssigkeit. Es kann auch ein Reservoir für die Flüssigkeit in geeignet aufbereiteter Form vorgesehen sein (nicht dargestellt).

## Patentansprüche

1. Verfahren zur Herstellung einer Oberflächenstruktur, insbesondere auf einem chirurgischen Implantat, durch Materialabtrag mittels eines Flüssigkeitsstrahls (1), der aus einer Düse (10) unter hohem Druck (p) abgegeben wird, wobei eine Abtragsstelle (3) auf einer zu strukturierenden Oberfläche (20a) eines Substrats (20) unter Erzeugung einer vorgegebenen Makrotopographie (2') oder einer weitgehend planen Oberfläche gesteuert bewegt wird, nämlich durch Bewegen der Düse und/oder des Substrats,
dadurch gekennzeichnet, dass die Flüssigkeit des Hochdruckstrahls (1) bei einem vorgegebenen Durchmesser d der Düse mit einem genügend hohen Druck p abgegeben wird, so dass durch den Materialabtrag eine lineare Spur (2) mit quasi-fraktaler Mikrotopographie (4) erzeugt wird, wobei die Spurbreite D mindestens doppelt so gross ist wie d und wobei für p und d Werte in folgenden Bereichen vorgesehen sind:
100 bar < p < 3000 bar und 0.1 mm < d < 10 mm;
oder p > 3000 bar und d > 0.03 mm.

2. Verfahren nach Anspruch 1, dadurch gekennzeichnet, dass als Flüssigkeit (1) ein Medium verwendet wird, das weitgehend frei von Feststoffpartikeln ist, und dass diese Flüssigkeit insbesondere Wasser ist, vorzugsweise geeignet aufbereitetes Wasser.

3. Verfahren nach Anspruch 1 oder 2, dadurch gekennzeichnet, dass eine Schar von Abtragsspuren (2) erzeugt wird, die lokal weitgehend parallel sind, wobei zwischen benachbarten Spuren jeweils ein gut erkennbarer Grat (22) oder ein praktisch nicht erkennbarer Grat sichtbar ist, oder dass eine nichtlineare Abtragsspur (8) erzeugt wird, deren Verlauf insbesondere mäanderförmig ist.

4. Verfahren nach Anspruch 3, dadurch gekennzeichnet, dass zwei oder mehr Scharen (29, 29', 29") von Spuren (2) in einer kreuzweisen Anordnung übereinander aufgebracht werden.

5. Verfahren nach einem der Ansprüche 1 bis 4, dadurch gekennzeichnet, dass der Abstand (a) zwischen einer Austrittsöffnung (11) der Düse (10) und der zu bearbeitenden Oberfläche (20a) rund 0.1 - 20 mm beträgt und dass der Winkel (β = 90° - α) zwischen dem Flüssigkeitsstrahl (1) und der erzeugten Abtragsspur (2) 90° oder vorzugsweise kleiner als 90° ist.

6. Verfahren nach einem der Ansprüche 1 bis 5, dadurch gekennzeichnet, dass in einem ersten oder zweiten Schritt ein weiteres Verfahren zu einer Oberflächenbehandlung, beispielsweise ein Sandstrahl- oder ein Beschichtungsverfahren, angewendet wird, wobei das Sandstrahlverfahren vorzugsweise in dem ersten Schritt angewendet wird.

7. Anlage zum Durchführen des Verfahrens gemäss einem der Ansprüche 1 bis 6, folgende Komponenten umfassend: Mittel (100, 202) zum Erzeugen einer Relativgeschwindigkeit (v) zwischen dem zu bearbeitenden Körper (200) und der Abtragsstelle, nämlich eine gesteuert bewegbare Halterung (100) für die Düse (10) und/oder eine gesteuert bewegbare Halterung (201) für den Körper, wobei entweder die Düse oder der Körper in einer raumfesten Position gehalten werden kann,
ferner eine elektronische, programmierbare Steuereinrichtung (102) für die Mittel (100, 202) zum Erzeugen der Relativgeschwindigkeit und eine Hochdruckpumpe (101) für eine Erzeugung des Flüssigkeitsstrahls (1).

8. Anlage nach Anspruch 7, dadurch gekennzeichnet, dass eine Einrichtung (103) zum Aufbereiten der Flüssigkeit, insbesondere zu einer Filtrierung und/oder Entmineralisierung der Flüssigkeit, vorgesehen ist oder dass ein Reservoir für die Flüssigkeit in geeignet aufbereiteter Form vorgesehen ist.

9. Anwendung des Verfahrens gemäss einem der Ansprüche 1 bis 5, dadurch gekennzeichnet, dass Schaftoberflächen von Gelenkprothesen, die vorzugsweise für eine zementlose Implantation vorgesehen sind, oder Oberflächen von Dentalimplantaten strukturiert werden, wobei die zu strukturierenden Oberflächen in einer Vor- oder Nachbehandlung mit einem das Knochenwachstum fördernden Material, insbesondere einer Calcium-Phosphor-Verbindung, beschichtet werden.

10. Anwendung des Verfahrens gemäss einem der Ansprüche 1 bis 5, dadurch gekennzeichnet, dass bei einer elektrophysiologischen Elektrode ein Pol an dessen Oberfläche strukturiert wird, um - dank einer grossen spezifischen Oberfläche - eine wirkungsvolle Energieübertragung von dem Pol auf ein physiologisch zu beeinflussendes Gewebe zu erhalten, wobei die Elektrode insbesondere Teil eines Herzschrittmachers oder eines Defibrillators ist.

11. Anwendung des Verfahrens gemäss einem der Ansprüche 1 bis 5, dadurch gekennzeichnet, dass Werkstoffe bearbeitet werden, nämlich zur
- Erzeugung von Strukturen an homogenen, quasiisotropen Werkstoffen, um beispielsweise eine Welligkeit oder einen Orangenhauteffekt zu erzielen, oder
- Strukturierung von inhomogenen Werkstoffen, wobei unterschiedliches Verhalten bezüglich Löslichkeit oder Erosion genutzt werden.
